# EUROPEAN PATENT APPLICATION

(11) **EP 3 508 214 A1**
(43) Date of publication of application: **10.07.2019**
(21) Application number: 17824332.5
(22) Date of filing: 06.07.2017
(51) Int. Cl.: A61K 38/42, A61K 47/62, A61P 9/10

(54) **THERAPEUTIC AGENT FOR ISCHEMIC DISEASES**

(30) Priority: 06.07.2016 JP 2016134478
(71) Applicant: Chuo University, Tokyo 192-0393 (JP)
(72) Inventor: KOMATSU Teruyuki, Tokyo 112-8551 (JP); FUNAKI Ryosuke, Tokyo 112-8551 (JP); ABUMIYA Takeo, Sapporo-shi Hokkaido 060-0808 (JP); GEKKA Masayuki, Sapporo-shi Hokkaido 060-0808 (JP); HOUKIN Kiyohiro, Sapporo-shi Hokkaido 060-0808 (JP)
(74) Representative: HGF Limited
(86) International application number: PCT/JP2017/024857
(87) International publication number: WO 2018/008729

(57) **Abstract**

Provided is a therapeutic agent for ischemic disease that exhibits a tissue-protecting effect against tissue damage associated with ischemia. The therapeutic agent includes, as an active ingredient, a hemoglobin-albumin complex in which albumin serving as a shell is bound to hemoglobin serving as a core via a crosslinker. The therapeutic agent is used in treatment of ischemic disease.

## Description

### TECHNICAL FIELD

The present disclosure relates to a therapeutic agent for ischemic disease.

### BACKGROUND

Ischemic diseases are responsible for a high proportion of deaths, with strokes, in particular, being the fourth highest disease among causes of death in Japan. In 60% of cases, the cause of death is cerebral infarction. Since 2010, the number of cases of cerebral infarction in Japan has exceeded 500,000 people per year.

The first point of action in cerebral infarction therapy is to relieve vascular occlusion. Vascular occlusion can be relieved by intravenous injection of tissue plasminogen activator (tPA) or removal of a thrombus inside a blood vessel. Although change associated with ischemia is reversible when blood flow is restored through these therapies so long as the time until recanalization is short, irreversible tissue damage occurs if the time until recanalization is long (refer to NPL 1). The time limit from onset to treatment is 4.5 hours in the case of tPA intravenous injection and 8 hours, at most, in the case of thrombus removal. Brain tissue becomes harder to save as reduction in cerebral blood flow worsens and the time until restoration of blood flow increases.

Restoration of blood flow may conversely exacerbate tissue damage by a phenomenon referred to as ischemia-reperfusion (I/R) injury. When blood reperfuses to tissue in an ischemic condition, this may elicit an inflammatory response or the like in microvessels, for example, in the reperfused region, breakdown of vascular walls may occur, and cerebral edema and hemorrhagic infarcts may be exacerbated. Consequently, not only is it necessary to deal with restoration of blood flow in cerebral infarction therapy, but also ischemia-reperfusion injury.

One mechanism that may cause ischemia-reperfusion injury is thought to be inflammatory response due to white blood cells (refer to NPL 2). When a white blood cell adheres to intercellular adhesion molecule-1 (ICAM-1) expressed at vascular endothelium, the white blood cell is activated and produces matrix metalloprotease-9 (MMP-9). MMP-9 is known to contribute to the breakdown of microvessels and lead to edema or hemorrhage. It has been confirmed through animal trials that infarction can be reduced by inhibiting activation of white blood cells. However, since inhibiting activation of white blood cells throughout the body may reduce immunity (i.e., may increase the risk of infection), further improvement is required.

Against this background, various neuroprotective therapies for alleviation of ischemia-reperfusion injury have been investigated. One example of such a method is neuroprotective therapy using an artificial oxygen carrier. Examples have been reported in which a fluorocarbon emulsion (refer to PTL 1), oxygen nanobubbles (refer to PTL 2), and liposome-encapsulated hemoglobin (LEH) (refer to NPL 3) have been used as artificial oxygen carriers.

The inventors have studied the administration of LEH solution that does not contain white blood cells into the internal carotid artery after recanalization of an occluded blood vessel with the aim of preventing white blood cells from flowing into a reperfused region. Moreover, the inventors have demonstrated in a rat transient middle cerebral artery occlusion (MCAO) model that ischemia-reperfusion injury can be reduced when LEH solution is administered into the internal carotid artery after recanalization of an occluded blood vessel (refer to NPL 4).

The development of artificial oxygen carriers as red blood cell substitutes has been progressing mainly in the United States, Europe, and Japan since the 1980s, and a large number of formulations have so far been evaluated. However, no formulation has yet been put into practical use despite the efforts made over almost half a century. In the United States, intramolecularly crosslinked hemoglobin in which human hemoglobin is intramolecularly crosslinked (for example, refer to PTL 3), hemoglobin polymer in which human hemoglobin is bound by a crosslinker (for example, refer to PTL 4), hemoglobin polymer in which bovine hemoglobin is bound by a crosslinker (for example, refer to PTL 5), PEG-modified hemoglobin in which the water-soluble polymer poly(ethylene glycol) (PEG) is bound to the molecular surface of human hemoglobin (for example, refer to PTL 6), and so forth have been developed, and clinical trials are being conducted. These artificial oxygen carriers are designed to avoid renal excretion accompanying dissociation of hemoglobin subunits by crosslinking between subunits or increasing molecule size (molecular weight). However, the artificial oxygen carriers that have been studied so far in clinical trials have, for example, caused elevated blood pressure through vascular constriction or not demonstrated a difference in effect between a group to which the artificial oxygen carrier is administered and a group to which saline is administered, and are yet to receive approval from the United States Food and Drug Administration (FDA), for example.

The inventors have synthesized a core-shell type hemoglobin-albumin complex in which albumin is bound to hemoglobin and have demonstrated that this hemoglobin-albumin complex can serve as a red blood cell substitute (refer to PTL 7). Albumin is the most abundant protein in plasma and has functions such as maintaining colloid osmotic pressure, and also transportation and storage of exogenous and endogenous substances. A hemoglobin-albumin complex having a structure in which the oxygen-transporting protein hemoglobin is encapsulated by albumin has a strong negative charge at the molecular surface thereof. This negative charge reduces leakage of the hemoglobin-albumin complex out of blood vessels and lengthens the residence time of the hemoglobin-albumin complex in the blood. Moreover, a hemoglobin-albumin complex functions as a safe red blood cell substitute due to its high biocompatibility (refer to NPL 5).

### CITATION LIST

### Patent Literature

PTL 1: JP H7-509397 A
PTL 2: JP 2011-001271 A
PTL 3: JP H10-306036 A
PTL 4: JP H11-502821 A
PTL 5: JP 2006-516994 A
PTL 6: JP 2005-515225 A
PTL 7: WO 2012/117688 A1

### Non-Patent Literature

NPL 1: Z. S. Shi et al., Stroke, 45 (7), 1977-1984 (2014)
NPL 2: G. J. del Zoppo et al., Stroke, 22, 1276-1283 (1991)
NPL 3: A. T. Kawaguchi et al., Artif. Organs, 33, 153-158 (2009)
NPL 4: D. Shimbo et al., Brain Res., 1554, 59-66 (2014)
NPL 5: R. Haruki et al., Sci. Rep., 5, 12778, 1-9 (2015)

### SUMMARY

### (Technical Problem)

An objective of the present disclosure is to provide a therapeutic agent for ischemic disease that displays a tissue-protecting effect against tissue damage associated with ischemia.

### (Solution to Problem)

A therapeutic agent for ischemic disease according to the present disclosure comprises, as an active ingredient, a hemoglobin-albumin complex in which albumin serving as a shell is bound to hemoglobin serving as a core via a crosslinker.

For the therapeutic agent for ischemic disease according to the present disclosure, the ischemic disease may preferably be at least one selected from the group consisting of ischemic cerebrovascular disease, ischemic heart disease, ischemic lung disease, ischemic nephropathy, ischemic hepatitis, and limb ischemia.

For the therapeutic agent for ischemic disease according to the present disclosure, the ischemic disease may preferably be cerebral infarction.

The therapeutic agent for ischemic disease according to the present disclosure may preferably be administered in order to deliver oxygen to tissue to which oxygen cannot be delivered by red blood cells.

The therapeutic agent for ischemic disease according to the present disclosure may preferably be used not in combination with treatment for relieving vascular occlusion or vascular constriction.

The therapeutic agent for ischemic disease according to the present disclosure may preferably be used in combination with treatment for relieving vascular occlusion or vascular constriction.

The therapeutic agent for ischemic disease according to the present disclosure may preferably be used to reduce ischemia-reperfusion injury through intravascular administration when recanalization therapy is performed or after ischemia reperfusion.

In a situation in which an individual who has undergone normal reperfusion and an individual who has been administered the therapeutic agent for ischemic disease according to the present disclosure in combination with reperfusion are compared in terms of cerebral infarct volume and cerebral edema volume, either or both of cerebral infarct volume and cerebral edema volume may preferably be significantly smaller for the individual who has been administered the therapeutic agent than for the individual who has undergone normal reperfusion.

In a situation in which an individual who has undergone normal reperfusion and an individual who has been administered the therapeutic agent for ischemic disease according to the present disclosure in combination with reperfusion are compared in terms of abundance of 4-HNE and expression of matrix metalloprotease-9 at a cerebral infarction site, either or both of abundance of 4-HNE and expression of matrix metalloprotease-9 may preferably be significantly lower for the individual who has been administered the therapeutic agent than for the individual who has undergone normal reperfusion.

In a situation in which an individual who has undergone normal reperfusion and an individual who has been administered the therapeutic agent for ischemic disease according to the present disclosure in combination with reperfusion are compared in terms of post-cerebral infarction neurological disorder, neurological disorder may preferably be significantly improved for the individual who has been administered the therapeutic agent relative to the individual who has undergone normal reperfusion.

A single dose of the therapeutic agent for ischemic disease according to the present disclosure to an individual may preferably be 100 mg to 1,000 mg, in terms of hemoglobin content, per 1 kg of body weight of the individual.

In the therapeutic agent for ischemic disease according to the present disclosure, the hemoglobin-albumin complex may preferably have an average particle diameter of 30 nm or less.

In the therapeutic agent for ischemic disease according to the present disclosure, a bonding site with the crosslinker in the hemoglobin of the hemoglobin-albumin complex may preferably be lysine, and a bonding site with the crosslinker in the albumin of the hemoglobin-albumin complex may preferably be cysteine 34.

In the therapeutic agent for ischemic disease according to the present disclosure, a bond between the hemoglobin and the crosslinker may preferably be an amide bond, and a bond between the albumin and the crosslinker may preferably be a sulfide bond or a disulfide bond.

In the therapeutic agent for ischemic disease according to the present disclosure, the number of molecules of the albumin in the hemoglobin-albumin complex may preferably be 1 to 7.

In the therapeutic agent for ischemic disease according to the present disclosure, the hemoglobin may preferably be at least one selected from the group consisting of human hemoglobin, bovine hemoglobin, pig hemoglobin, horse hemoglobin, dog hemoglobin, cat hemoglobin, rabbit hemoglobin, recombinant human hemoglobin, and intramolecularly crosslinked hemoglobin.

In the therapeutic agent for ischemic disease according to the present disclosure, the albumin may preferably be at least one selected from the group consisting of human serum albumin, bovine serum albumin, pig serum albumin, horse serum albumin, dog serum albumin, cat serum albumin, rabbit serum albumin, recombinant human serum albumin, recombinant bovine serum albumin, recombinant dog serum albumin, and recombinant cat serum albumin.

In the therapeutic agent for ischemic disease according to the present disclosure, the crosslinker may preferably be at least one selected from the group consisting of compounds represented by general formulae (1) to (3) and chemical formula (1), shown below, 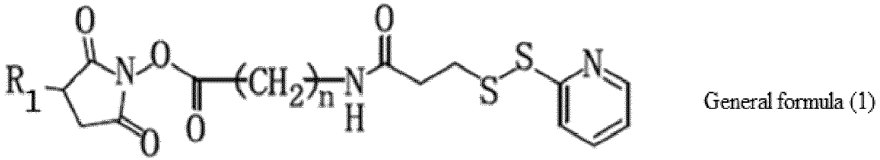 where, in general formula (1), R₁ represents a hydrogen atom or SO₃⁻Na⁺ and n represents an integer of 1 to 10, 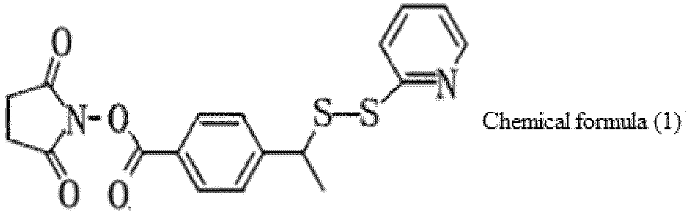 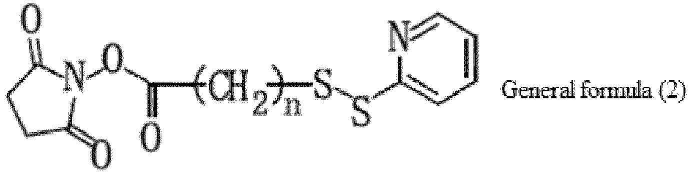 in general formula (2), n represents an integer of 1 to 10, 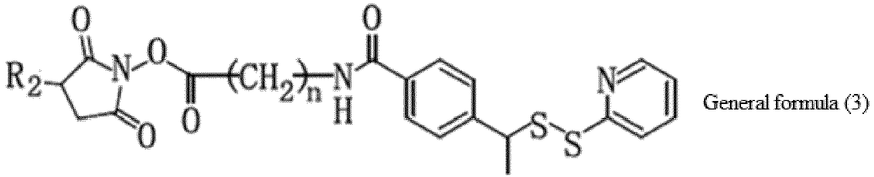 and in general formula (3), R₂ represents a hydrogen atom or SO₃⁻Na⁺ and n represents an integer of 1 to 10.

In the therapeutic agent for ischemic disease according to the present disclosure, the crosslinker may preferably be a compound represented by general formula (4), shown below, 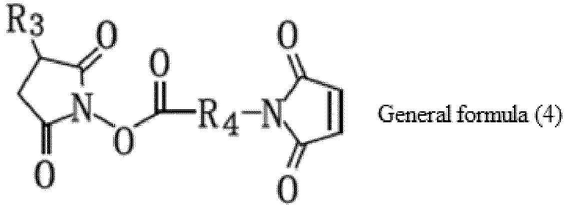 where, in general formula (4), R₃ represents a hydrogen atom or SO₃⁻Na⁺ and R₄ represents any one of general formulae (5) and (6) and chemical formulae (2) to (4), shown below, 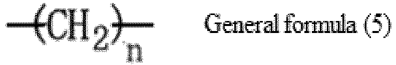 in general formula (5), n represents an integer of 1 to 10, 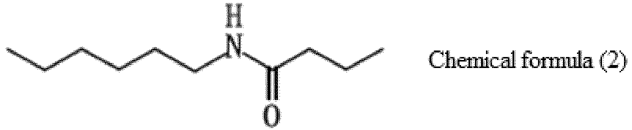 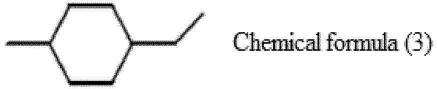 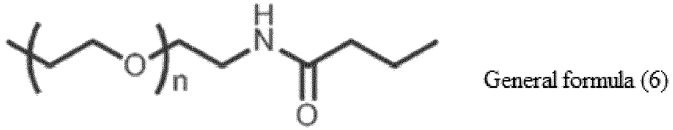 and in general formula (6), n represents an integer of 2, 4, 6, 8, 10, or 12,

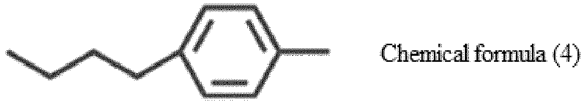

A method according to the present disclosure comprises treating ischemic disease by administering, to a patient, a hemoglobin-albumin complex in which albumin serving as a shell is bound to hemoglobin serving as a core via a crosslinker.

A hemoglobin-albumin complex according to the present disclosure is a hemoglobin-albumin complex in which albumin serving as a shell is bound to hemoglobin serving as a core via a crosslinker, and that is used as a therapeutic agent for ischemic disease.

A use according to the present disclosure comprises using a hemoglobin-albumin complex in which albumin serving as a shell is bound to hemoglobin serving as a core via a crosslinker for producing a therapeutic agent for ischemic disease.

### (Advantageous Effect)

According to the present disclosure, it is possible to provide a therapeutic agent for ischemic disease that displays a tissue-protecting effect against tissue damage associated with ischemia.

### BRIEF DESCRIPTION OF THE DRAWINGS

In the accompanying drawings:
FIG. 1 illustrates one embodiment of a hemoglobin-albumin complex contained in a therapeutic agent according to the present disclosure;
FIG. 2 is a bar graph illustrating the degree of neurological disorder in rats 24 hours after reperfusion, wherein the vertical axis indicates a score for the degree of neurological disorder;
FIG. 3A shows photographs of TTC-stained brain sections of rats 24 hours after reperfusion;
FIG. 3B is a bar graph illustrating cerebral infarct volume (%) determined from serial section cerebral infarct area of TTC-stained brain sections of rats 24 hours after reperfusion;
FIG. 3C is a bar graph illustrating cerebral edema volume (%) determined from serial section cerebral edema area of TTC-stained brain sections of rats 24 hours after reperfusion;
FIG. 4A shows photographs taken in measurement of 4-HNE abundance and β-actin expression by western blotting using rat brains 24 hours after reperfusion as samples;
FIG. 4B is a bar graph illustrating a ratio of 4-HNE abundance relative to β-actin expression;
FIG. 5A shows photographs taken in measurement of MMP-9 and β-actin expression by western blotting using rat brains 24 hours after reperfusion as samples;
FIG. 5B is a bar graph illustrating a ratio of MMP-9 expression relative to β-actin expression;
FIG. 6 shows photographs of reperfused regions of rat brains during a hyperacute phase of reperfusion that have been immunostained using anti-human albumin antibody or anti-rat red blood cell antibody;
FIG. 7 is a bar graph illustrating the number of microvessels that are antibody positive in reperfused regions of rat brains during a hyperacute phase of reperfusion;
FIG. 8 illustrates a method of measuring microvessel diameter and measuring a difference between perivascular space and microvessel diameter as a proportion relative to microvessel diameter;
FIG. 9 shows photographs in which microvessels in reperfused regions of rat brains during a hyperacute phase of reperfusion have been immunostained;
FIG. 10 shows a line graph illustrating a difference between perivascular space and microvessel diameter as a proportion relative to microvessel diameter and a line graph illustrating microvessel diameter in reperfused regions of rat brains during a hyperacute phase of reperfusion; and
FIG. 11 shows a line graph illustrating cerebral blood flow and a line graph illustrating brain tissue oxygen partial pressure in reperfused regions of rat brains during a hyperacute phase of reperfusion.

### DETAILED DESCRIPTION

The following provides an illustrative description of one embodiment of the present disclosure.

### (Therapeutic agent)

A therapeutic agent for ischemic disease (hereinafter, also referred to simply as a "therapeutic agent") according to the present disclosure contains, as an active ingredient, a hemoglobin-albumin complex in which albumin serving as a shell is bound to hemoglobin serving as a core via a crosslinker.

The therapeutic agent according to the present disclosure contains the hemoglobin-albumin complex as an active ingredient and may further contain other ingredients as necessary.

### <Ischemia>

Ischemia is a condition in which thrombosis, embolism, angiostenosis, or the like causes occlusion or constriction of a blood vessel, and thereby reduces the blood supply to a body organ, tissue, or other site. The occurrence of ischemia may lead to direct ischemic injury (i.e., tissue damage caused by reduced oxygen supply).

### <Ischemic disease>

The aforementioned ischemic disease can be selected as appropriate depending on the objective without any specific limitations and examples thereof include ischemic cerebrovascular disease, ischemic heart disease, ischemic lung disease, ischemic nephropathy, ischemic hepatitis, and limb ischemia.

Examples of the etiology of the ischemic disease include mainly (i) occlusion and/or constriction of a blood vessel, (ii) direct tissue damage resulting therefrom, and (iii) ischemia-reperfusion injury occurring after blood flow is restored in reperfusion. Dysfunction of organs occurs in ischemic disease through these etiologies.

The therapeutic agent according to the present disclosure displays a tissue-protecting effect against direct tissue damage and ischemia-reperfusion injury among these etiologies, and, in particular, has a high tissue-protecting effect against ischemia-reperfusion injury.

### <<Ischemia reperfusion>>

Ischemia reperfusion is the restoration of blood supply to tissue in which blood supply has decreased and ischemia has occurred. This reperfusion can be carried out through relief of vascular occlusion or vascular constriction. The reperfusion can restore viable ischemic tissue and thereby inhibit necrosis. However, the reperfusion itself may cause further injury of ischemic tissue.

Relief of vascular occlusion or vascular constriction can be performed through treatment such as administration of tissue plasminogen activator (tPA) via a drip, surgery to remove a thrombus or infarct, or surgery to introduce a stent or the like.

### -Ischemia-reperfusion injury-

The term "ischemia-reperfusion injury" as used in the present description refers to tissue damage that occurs when there is reperfusion of blood flow to ischemic tissue after relief of vascular occlusion and/or constriction.

Ischemia-reperfusion injury in ischemic disease often becomes serious in the brain or the heart, but is not limited to these organs and may also occur by the same mechanism in various organs such as the lungs, kidneys, and limbs.

### <<Cerebral infarction>>

Cerebral infarction refers to a condition in which occlusion and/or constriction of a cerebral blood vessel causes ischemia, leading to necrosis or near necrosis of brain tissue.

Examples of the etiology of the cerebral infarction include mainly (i) occlusion and/or constriction of a cerebral blood vessel, (ii) direct tissue damage resulting therefrom, and (iii) ischemia-reperfusion injury occurring after restoration of blood flow in reperfusion. Dysfunction of the brain occurs in cerebral infarction through these etiologies.

The therapeutic agent according to the present disclosure displays a tissue-protecting effect against direct tissue damage and ischemia-reperfusion injury among these etiologies, and, in particular, has a high tissue-protecting effect against ischemia-reperfusion injury.

The individual for whom the therapeutic agent is used may, for example, be a patient who has been diagnosed as having ischemic disease, a patient who is undergoing treatment for ischemic disease, or a healthy patient for whom specific symptoms have not been identified but is suspected to have ischemic disease.

Moreover, the individual for whom the therapeutic agent is used is not limited to a human and may be another mammal such as a mouse, a rat, a monkey, a rabbit, a dog, a cat, or a horse.

The type of albumin is preferably selected in accordance with the type of animal for which the therapeutic agent is to be used. When the albumin in the hemoglobin-albumin complex is albumin originating from the same species of animal, the hemoglobin-albumin complex is unlikely to be identified as a heterologous antigen in the body of an individual to whom the hemoglobin-albumin complex is administered, and an immune response against the hemoglobin-albumin complex can be avoided.

### <<Method of administration>>

The method by which the therapeutic agent is administered to an individual can be selected as appropriate depending on the objective without any specific limitations and examples thereof include administration into a vein and administration into an artery. One of these administration methods may be used individually, or two or more of these administration methods may be used in combination.

In a case in which a site of occlusion or constriction is known prior to administration of the therapeutic agent, the therapeutic agent may be administered directly into a blood vessel where there is occlusion or constriction so as to enable more efficient flow of the hemoglobin-albumin complex into ischemic tissue.

The therapeutic agent is preferably administered in order to deliver oxygen to tissue to which oxygen cannot be delivered by red blood cells. This enables oxygen delivery to tissue at a distal side from a blood vessel even when the blood vessel is occluded or constricted, and can thereby reduce the etiology of direct tissue damage described above.

The therapeutic agent may be used not in combination with treatment for relieving vascular occlusion or vascular constriction, or may be used in combination with treatment for relieving vascular occlusion or vascular constriction.

Advantages to using the therapeutic agent not in combination with treatment for relieving vascular occlusion or vascular constriction include an advantage that treatment for reducing tissue damage with respect to a patient suffering from ischemic disease that requires emergency treatment can be carried out through prompt administration of the therapeutic agent and an advantage that treatment for reducing tissue damage can be carried out through administration of the therapeutic agent in a case in which it is difficult to relieve vascular occlusion or vascular constriction. Note that the case in which it is difficult to relieve vascular occlusion or vascular constriction may, for example, be a case in which treatment to relieve vascular occlusion or vascular constriction cannot be carried out or a case in which it is anticipated that sufficient fibrinolysis of a thrombus will not proceed even if a drug that promotes fibrinolysis is administered.

Advantages of using the therapeutic agent in combination with treatment for relieving vascular occlusion or vascular constriction include an advantage that in addition to relieving vascular occlusion or vascular constriction, ischemia-reperfusion injury that may result from relief of vascular occlusion or vascular constriction can also be effectively reduced.

The hemoglobin-albumin complex contained in the therapeutic agent has a size that is roughly 1/800^{th} of the diameter of a red blood cell and can, therefore, flow to sites where red blood cells cannot flow when vascular constriction occurs. Consequently, the hemoglobin-albumin complex can flow into ischemic tissue even when a blood vessel is constricted and has an effect of reducing tissue damage. Accordingly, the therapeutic agent can reduce tissue damage even when administered not in combination with treatment for relieving vascular occlusion or vascular constriction.

When the therapeutic agent is administered in combination with treatment for relieving vascular occlusion or vascular constriction, ischemia-reperfusion injury can be significantly reduced compared to a case in which only treatment for relieving vascular occlusion or vascular constriction is carried out.

Therefore, when the therapeutic agent according to the present disclosure is administered in combination with treatment for relieving vascular occlusion or vascular constriction, relief of occlusion or constriction and reduction of ischemia-reperfusion injury can both be effectively achieved.

### <<Dose>>

A single dose of the therapeutic agent to an individual can be selected as appropriate depending on the objective without any specific limitations. The dose is preferably set such that hemoglobin content is 100 mg to 1,000 mg per 1 kg of body weight of the individual, and more preferably 200 mg to 700 mg per 1 kg of body weight of the individual.

A dose of 100 mg or more in terms of hemoglobin content is preferable from a viewpoint of further reducing ischemia-reperfusion injury, whereas a dose of 1,000 mg or less in terms of hemoglobin content is preferable from a viewpoint of cost efficiency. A dose within the more preferable range set forth above is more advantageous for the same reasons.

The therapeutic agent may be administered once a day, or may be split between multiple administrations. Moreover, the dose may be determined as appropriate for each individual case in consideration of symptoms, age, sex, and so forth. Administration of the therapeutic agent may be carried out continuously or intermittently.

In a situation in which an individual who has undergone normal reperfusion and an individual who has been administered the therapeutic agent according to the present disclosure in combination with reperfusion are compared in terms of cerebral infarct volume and cerebral edema volume, either or both of cerebral infarct volume and cerebral edema volume are significantly smaller for the individual who has been administered the therapeutic agent according to the present disclosure than for the individual who has undergone normal reperfusion.

Moreover, in a situation in which an individual who has undergone normal reperfusion and an individual who has been administered the therapeutic agent according to the present disclosure in combination with reperfusion are compared in terms of abundance of 4-HNE and expression of matrix metalloprotease-9 at a cerebral infarction site, either or both of abundance of 4-HNE and expression of matrix metalloprotease-9 are significantly lower for the individual who has been administered the therapeutic agent according to the present disclosure than for the individual who has undergone normal reperfusion.

Furthermore, in a situation in which an individual who has undergone normal reperfusion and an individual who has been administered the therapeutic agent according to the present disclosure in combination with reperfusion are compared in terms of post-cerebral infarction neurological disorder, neurological disorder is significantly improved for the individual who has been administered the therapeutic agent according to present disclosure relative to the individual who has undergone normal reperfusion.

Note that normal reperfusion refers to treatment for relieving vascular occlusion or vascular constriction such as administration of tissue plasminogen activator (tPA) via a drip, surgery to remove a thrombus or infarct, or surgery to introduce a stent or the like such as previously described.

Cerebral infarct volume and cerebral edema volume are morphological indicators for evaluating ischemic injury to the brain. 4-HNE (4-hydroxy-2-nonenal) is a product of oxidative stress and the abundance thereof serves as an indicator of oxidation condition in tissue. Matrix metalloprotease-9 (MMP-9) is a protease that is mainly produced by white blood cells and the expression thereof serves as an indicator of inflammation condition in tissue. Post-cerebral infarction neurological disorder serves as an indicator of the state of brain function in an individual after cerebral infarction.

One reason that the therapeutic agent according to the present disclosure can reduce ischemia-reperfusion injury and display a tissue-protecting effect in ischemic disease is thought to be that the hemoglobin-albumin complex is an extremely small artificial oxygen carrier, which enables efficient oxygen transport even in a microvessel in which the lumen has narrowed such as observed in ischemia-reperfusion injury. Administration of the therapeutic agent according to the present disclosure is thought to inhibit damage of endothelial cells and thereby suppress expression of ICAM-1 by vascular endothelial cells. As a result, adhesion of patrolling white blood cells can be inhibited, and activation of white blood cells can be avoided. Moreover, it is thought that degrading enzyme secretion by white blood cells can be inhibited and a protective effect can be displayed toward nerves, blood vessels, and other tissues.

### <Hemoglobin-albumin complex>

The hemoglobin-albumin complex includes hemoglobin serving as a core and albumin serving as a shell that is bound to the hemoglobin via a crosslinker, and may further include other parts as necessary.

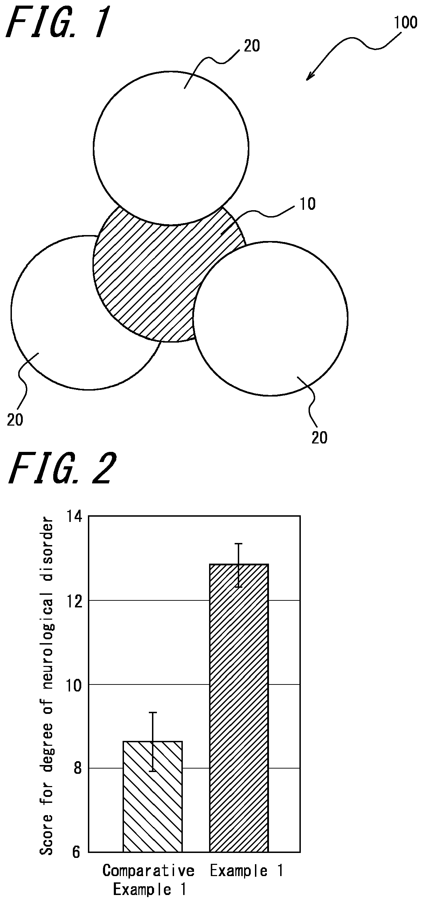

For example, the hemoglobin-albumin complex (star-shaped heterocluster) 100 contained in the therapeutic agent according to the present disclosure may include hemoglobin 10 as a core and three molecules of albumin 20 as a shell as illustrated in FIG. 1. The hemoglobin 10 and the albumin 20 in FIG. 1 are bound via a crosslinker (not illustrated).

In a case in which the hemoglobin-albumin complex includes three molecules of albumin bound to one molecule of hemoglobin via a crosslinker, the molecule size of the hemoglobin-albumin complex is approximately 10 nm. This size is approximately 1/800^{th} of the diameter of a human red blood cell (approximately 8 µm) and is approximately 1/20^{th} of the diameter of the previously described LEH (200 nm to 250 nm). The extremely small size of the hemoglobin-albumin complex allows the hemoglobin-albumin complex to flow into regions where red blood cells and LEH are not physically able to flow.

Although the molecule size of the hemoglobin-albumin complex is approximately 10 nm in a case in which three molecules of albumin are bound to one molecule of hemoglobin via the crosslinker, the size of the hemoglobin-albumin complex can be increased through an increase in the number of molecules of albumin that are bound or through further bonding of a component other than albumin.

The average particle diameter of the hemoglobin-albumin complex is preferably 30 nm or less, and more preferably 20 nm or less.

It is advantageous for the average particle diameter of the hemoglobin-albumin complex to be 30 nm or less in terms that the hemoglobin-albumin complex can flow into even small gaps, and it is more advantageous for the average particle diameter to be 20 nm or less for the same reason.

The isoelectric point of the hemoglobin-albumin complex can be adjusted as appropriate depending on the objective without any specific limitations and is preferably 4.0 to 6.5, and more preferably 4.2 to 5.5. As a result of the isoelectric point of the hemoglobin-albumin complex being lower than 7, the molecular surface of the hemoglobin-albumin complex is negatively charged under physiological conditions. Consequently, it is difficult for the hemoglobin-albumin complex to leak out of blood vessels due to electrostatic repulsion with a basement membrane (negatively charged) at the outside of vascular endothelial cells. Therefore, the hemoglobin-albumin complex functions as a safe red blood cell substitute without renal excretion or side effects such as elevated blood pressure associated with leakage of the hemoglobin-albumin complex from blood vessels.

The hemoglobin serves as an oxygen binding site in the hemoglobin-albumin complex. This enables the hemoglobin-albumin complex to form a stable oxygenated product and efficiently supply oxygen to body tissue. Moreover, the hemoglobin-albumin complex is thought to have high biocompatibility and good metabolism because hemoglobin and albumin are biological substances. Furthermore, the hemoglobin-albumin complex has a clear three-dimensional structure even though it is comparatively easy to produce.

Since hemoglobin serves as the oxygen binding site in the hemoglobin-albumin complex, the hemoglobin-albumin complex has an S-shaped oxygen binding/dissociation curve, and an effect of improving oxygen transport capability is expected, in particular, in a case in which oxygen partial pressure in peripheral cells has fallen.

As a consequence of a structure being adopted in which the albumin covers the hemoglobin, the type of albumin tends to influence immune response in the body of an individual to which the therapeutic agent is administered. Therefore, the albumin of the hemoglobin-albumin complex is preferably albumin originating from the same species as the animal to which the therapeutic agent is to be administered.

On the other hand, the type of hemoglobin tends not to influence immune response in the body of an individual to which the therapeutic agent is administered because the hemoglobin is covered by the albumin. Therefore, no specific limitations are placed on the animal from which the hemoglobin originates from a viewpoint of immune response.

The albumin and the hemoglobin in the hemoglobin-albumin complex may originate from different animals to one another.

### <<Hemoglobin>>

A molecule of the hemoglobin is composed of four subunits that each include one protoheme. Oxygen bonds to an iron atom in the protoheme. In other words, four molecules of oxygen bond to one molecule of hemoglobin.

The hemoglobin can be selected as appropriate depending on the objective without any specific limitations other than being hemoglobin of a vertebrate. Examples of hemoglobin that may be used from a viewpoint of ease of acquisition include human hemoglobin, bovine hemoglobin, pig hemoglobin, horse hemoglobin, dog hemoglobin, cat hemoglobin, rabbit hemoglobin, recombinant human hemoglobin, and intramolecularly crosslinked hemoglobin. One of these types of hemoglobin may be used individually, or two or more of these types of hemoglobin may be used in combination.

Moreover, the hemoglobin may, for example, be hemoglobin obtained through dissolution of red blood cells, recombinant hemoglobin obtained by gene recombination technology, or intramolecularly crosslinked hemoglobin obtained through intramolecular crosslinking of subunits of such hemoglobin by a crosslinker. One of these types of hemoglobin may be used individually, or two or more of these types of hemoglobin may be used in combination.

Of these types of hemoglobin, bovine hemoglobin, pig hemoglobin, and horse hemoglobin, and particularly bovine hemoglobin and pig hemoglobin are advantageous in terms of enabling large scale production of the hemoglobin-albumin complex since they are easy to acquire from a viewpoint of supply volume as hemoglobin of industrially farmed animals.

### «Albumin»

The albumin is a simple protein having a principal function of regulating colloid osmotic pressure in blood, and also having a function as a transport protein for nutritive substances, drugs, and so forth. The albumin also has a pH buffering effect, esterase activity, and so forth. Moreover, since the albumin is a plasma protein, it is highly advantageous for use in organisms, and particularly as a red blood cell substitute.

The albumin has an isoelectric point of lower than 7 and has a strongly negatively charged molecular surface under physiological conditions. Consequently, it is difficult for the hemoglobin-albumin complex having the albumin as a shell to leak out of blood vessels due to electrostatic repulsion with a basement membrane (negatively charged) at the outside of vascular endothelial cells.

The albumin can be selected as appropriate depending on the objective without any specific limitations. Examples of albumin that may be used from a viewpoint of ease of acquisition include human serum albumin, bovine serum albumin, pig serum albumin, horse serum albumin, dog serum albumin, cat serum albumin, rabbit serum albumin, recombinant human serum albumin, recombinant bovine serum albumin, recombinant dog serum albumin, and recombinant cat serum albumin. One of these types of albumin may be used individually, or two or more of these types of albumin may be used in combination.

The number of molecules of albumin that are bound to the hemoglobin via the crosslinker can be selected as appropriate depending on the objective without any specific limitations and is preferably 1 to 7. Bonding of 8 or more molecules of albumin is thought to be difficult due to steric hinderance. A case in which the number of molecules of albumin is 2 to 5 is advantageous in terms that the hemoglobin can be sufficiently surrounded by the albumin. The number of molecules of hemoglobin included in the hemoglobin-albumin complex can be selected as appropriate depending on the objective without any specific limitations and is preferably 1.

The number of molecules of albumin may be measured by a method that is selected as appropriate depending on the objective without any specific limitations and examples of such methods include (1) a method in which calculation is performed based on the molecular weight of the entire hemoglobin-albumin complex measured by electrophoresis and the molecular weights of hemoglobin and albumin; (2) a method in which calculation is performed based on hemoglobin concentration calculated through quantification of heme by a cyanomethemoglobin method (for example, Alfresa Pharma Corporation, Nescauto Hemo Kit N, No. 138016-14) and protein concentration calculated through quantification of protein using a 660 nm method (for example, Pierce 660 nm Protein Assay Kit, Thermo Fisher Scientific); and (3) a method of observation using an electron microscope.

A hemoglobin-albumin complex for which the number of albumin molecules is a specific number can be isolated from a mixture of hemoglobin-albumin complexes having different numbers of albumin molecules by a method that is selected as appropriate depending on the objective without any specific limitations such as, for example, isolation by column chromatography (for example, gel filtration chromatography, ion exchange chromatography, affinity chromatography, or hydrophobic chromatography).

### <<Crosslinker>>

The crosslinker can be selected as appropriate depending on the objective without any specific limitations other than being a bifunctional crosslinker that can link hemoglobin and albumin. Examples of crosslinkers that can be used include compounds represented by the following general formulae (1) to (4) and chemical formula (1). One of these crosslinkers may be used individually, or two or more of these crosslinkers may be used in combination.

Of these crosslinkers, an α-(N-succinimidyl)-ω-pyridyldithio crosslinker (compound for which R₁ in general formula (1) is a hydrogen atom and n in general formula (1) is 5) and an α-(N-succinimidyl)-ω-maleimide crosslinker (compound for which R₃ in general formula (4) is a hydrogen atom and R₄ in general formula (4) is general formula (5); compound for which R₃ in general formula (4) is a hydrogen atom, R₄ in general formula (4) is general formula (5), and n = 5 or n = 2; and compound for which R₃ in general formula (4) is a hydrogen atom and R₄ in general formula (4) is chemical formula (3)) are preferable. 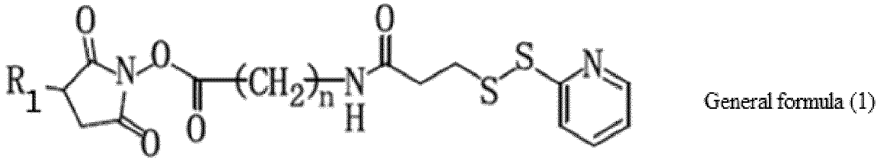 In general formula (1), R₁ represents a hydrogen atom or SO₃⁻Na⁺ and n represents an integer of 1 to 10. Typical examples include a compound for which n = 5. 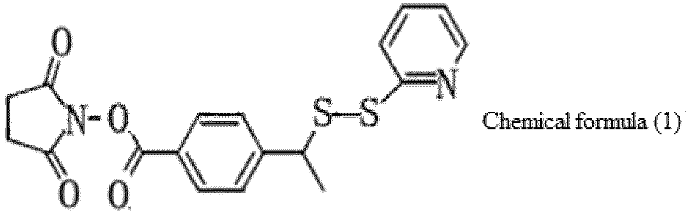 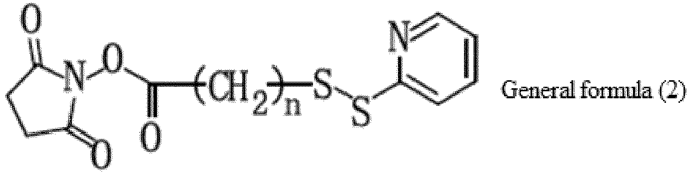 In general formula (2), n represents an integer of 1 to 10. Typical examples include a compound for which n = 2. 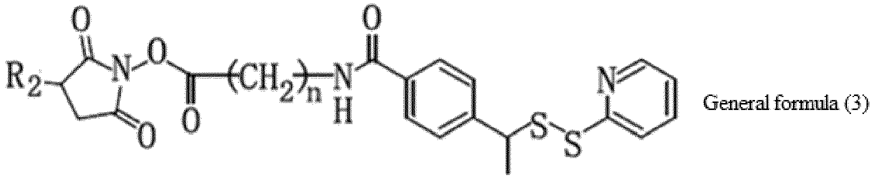 In general formula (3), R₂ represents a hydrogen atom or SO₃⁻Na⁺ and n represents an integer of 1 to 10. Typical examples include a compound for which n = 5. 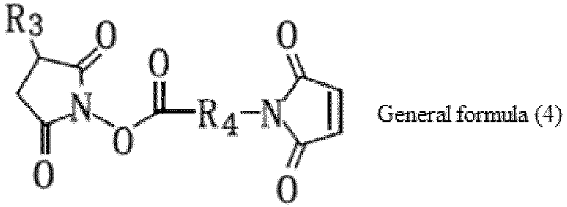 In general formula (4), R₃ represents a hydrogen atom or SO₃⁻Na⁺ and R₄ represents any one of the following general formulae (5) and (6) and chemical formulae (2) to (4). 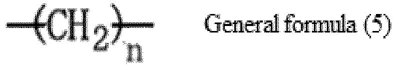 In general formula (5), n represents an integer of 1 to 10. 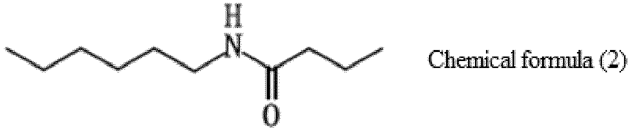 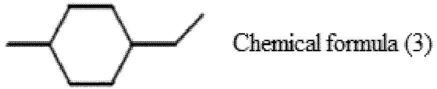 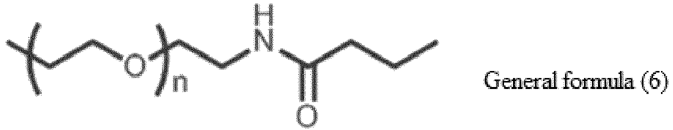 In general formula (6), n represents an integer of 2, 4, 6, 8, 10, or 12.

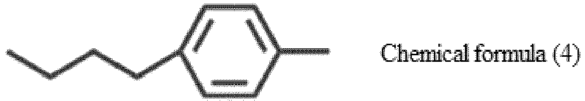

A succinimidyl group in the crosslinker and an amino group (-NH₂) of lysine in hemoglobin form an amide bond (covalent bond).

The method by which the amide bond is formed may, for example, be a method of stirring the hemoglobin and the crosslinker at 4°C to 30°C for 0.2 hours to 8 hours.

A pyridyldithio group in the crosslinker and cysteine 34 (reduced cysteine) in an albumin molecule form a new disulfide bond (covalent bond) through an exchange reaction. Note that this disulfide bond is easily broken.

The method by which the disulfide bond is formed may, for example, be a method of stirring the albumin and the crosslinker at 4°C to 30°C for 1 hour to 40 hours.

A maleimide group in the crosslinker and cysteine 34 (reduced cysteine) in a molecule of albumin form a sulfide bond (covalent bond).

The method by which the sulfide bond is formed may, for example, be a method of stirring the albumin and the crosslinker at 4°C to 30°C for 1 hour to 72 hours.

Since only one cysteine 34 (reduced cysteine) is present in an albumin molecule, the hemoglobin-albumin complex contained as an active ingredient in the therapeutic agent according to the present disclosure adopts a star-shaped cluster structure (for example, the structure illustrated in FIG. 1) and has a clear molecular structure.

### <<Other parts>>

Examples of other parts that can be selected as appropriate depending on the objective without any specific limitations include poly(ethylene glycol) introduced at the surface of the albumin through a covalent bond and protein bound to the hemoglobin together with the albumin.

### <<Dosage form of therapeutic agent>>

The dosage form of the therapeutic agent set forth above can be selected as appropriate depending on the objective without any specific limitations. For example, the dosage form of the therapeutic agent may be a liquid form or freeze dried dosage form. In the case of a freeze dried dosage form, the therapeutic agent may be dissolved or suspended in a sterile solvent such as sterile water prior to use.

The therapeutic agent is preferably sterilized through filtration using a filter, heating, compounding of a germicide, or energy ray irradiation.

### <<Other ingredients>>

Other ingredients that may be contained in the therapeutic agent in addition to the hemoglobin-albumin complex can be selected as appropriate depending on the objective without any specific limitations and examples thereof include aqueous or non-aqueous solutions, pH buffering agents, tonicity agents, preservatives, stabilizers, emulsifiers, dispersants, and local anesthetics. One of these other ingredients may be used individually, or two or more of these other ingredients may be used in combination.

### <<Concentration of hemoglobin-albumin complex>>

The concentration of the hemoglobin-albumin complex in the therapeutic agent can be selected as appropriate depending on the objective without any specific limitations. In a case in which the therapeutic agent is in a liquid form, the hemoglobin content is preferably 30 mg/mL to 70 mg/mL, and more preferably 40 mg/mL to 60 mg/mL.

### EXAMPLES

The following provides a more detailed description of the present disclosure through examples. However, the present disclosure is not in any way limited by the following examples and alterations may be made as appropriate without changing the essence thereof.

### (Example 1 and Comparative Example 1)

### (1) Production of therapeutic agent

### -Production Example 1: Production of bovine hemoglobin-crosslinker conjugate (Hb-SMCC)-

A recovery flask (300 mL capacity) was charged with 120 mL of a phosphate buffered saline (PBS) (pH 7.4) solution (0.1 mM) of bovine CO hemoglobin (Hb), was stirred (300 rpm) using a stirrer while 12 mL of a dimethyl sulfoxide (DMSO) solution (20 mM) of succinimidyl-4-(N-maleimidomethyl)cyclohexane-1-carboxylate (SMCC, Wako Pure Chemical Industries, Ltd.) was slowly added (SMCC/Hb = 20 [mol/mol]), and was then stirred (stirring speed: 100 rpm) in the dark at 4°C for 6 hours. The resultant reaction solution was filtered using a DISMIC filter (DISMIC-25CS; pore diameter: 0.2 µm) and was subsequently subjected to gel filtration chromatography (Sephadex G-25 Superfine, 5.0 cmØ, eluent: PBS (pH 7.4)) to remove unreacted crosslinker. The resultant PBS (pH 7.4) solution of a bovine hemoglobin-crosslinker conjugate (Hb-SMCC) was concentrated to 120 mL using a centrifugal ultrafiltration device (Vivaspin 20, cutoff molecular weight: 10 kDa) to achieve a concentration of 0.1 mM.

### -Production Example 2: Production of bovine hemoglobin-human serum albumin complex (Hb-HSA₃)SMCC-

A recovery flask (500 mL capacity) was charged with 120 mL of a PBS (pH 7.4) solution (1.0 mM) of human serum albumin (HSA), was stirred (300 rpm) using a stirrer while 120 mL of the PBS (pH 7.4) solution (0.1 mM) of the bovine hemoglobin-crosslinker conjugate (Hb-SMCC) was slowly added dropwise (HSA/Hb-SMCC = 10 [mol/mol]), and was then stirred (stirring speed: 100 rpm) in the dark at 4°C for 16 hours.

Gel filtration chromatography (GFC) was used to remove only unreacted HSA from the resultant reaction solution in order to obtain a bovine hemoglobin-human serum albumin complex. Specifically, the resultant reaction solution was filtered using a DISMIC filter (DISMIC-25CS; pore diameter: 0.2 µm) and passed through a gel filtration chromatograph (Superdex 200pg, 1 L, eluent: PBS (pH 7.4)) connected to a UV detector (280 nm) and a recorder in order to recover high molecular weight content other than HSA. The solution was also concentrated as necessary using a centrifugal ultrafiltration device (Vivaspin; cutoff molecular weight: 30 kDa). The Hb concentration was quantified by a cyanomethemoglobin method (Nescauto Hemo Kit N, Alfresa Pharma Corporation) and the protein concentration was quantified by a 660 nm method (Pierce 660 nm Protein Assay Kit, Thermo Fisher Scientific). The HSA/Hb ratio was 3.0, which indicates that a bovine hemoglobin-human serum albumin complex [[(Hb-HSA₃)SMCC] in which an average of three molecules of HSA were bound to Hb was obtained.

### -Production Example 3: Production of therapeutic agent A-

A therapeutic agent A was produced by dissolving the obtained hemoglobin-albumin complex (Hb-HSA₃)SMCC in PBS (pH 7.4) such that [Hb] = 5 g/dL. Note that in administration to rats, therapeutic agent that had been heated to 37°C was used.

### (2) Ischemia reperfusion test

A transient middle cerebral artery occlusion (MCAO) model typically used as a cerebral infarction model was used in order to perform an ischemia reperfusion test.

Eight to nine week-old rats (male Sprague-Dawley Rats (SD Rats) weighing 280 g to 320 g) were each anesthetized by isoflurane inhalation. A filament embolus (silicone-coated 4-0 nylon filament) was inserted from the internal carotid artery, was advanced upward until the origin of the middle cerebral artery was occluded, and was secured in place to create an ischemic condition in the middle cerebral artery region. Reduction in cerebral blood flow in ischemia was checked using a laser blood flowmeter (FLO-C1, Omegawave). Note that only rats for which the blood flow had decreased to 30% of the former value were used in subsequent experimentation. After two hours of ischemia, each of the rats was anesthetized once again, and the filament embolus was withdrawn to create a reperfusion condition. In the reperfusion, a tube (PE10 tube) for infusion was newly inserted into the internal carotid artery into which the filament embolus had been inserted, and 80 mL/kg/h of the therapeutic agent A was intra-arterially administered for 5 minutes to the region in which ischemia had occurred (Example 1, hemoglobin-albumin complex-administered group). A group for which the filament embolus was withdrawn without administration of the therapeutic agent A was used as a control group (Comparative Example 1, control group).

The degree of neurological disorder was evaluated 24 hours after reperfusion. Thereafter, the rats were euthanized and their brains were excised in order to perform pathological evaluation and molecular biological evaluation.

### (3) Evaluation of degree of neurological disorder

The degree of neurological disorder was evaluated using the method of Garcia et al. (Stroke, 26, 627-634 (1995)). Improvement of symptoms was evaluated by grading activity, walking condition, limb movement, and so forth using an 18-point scale (maximum of 18 points for normal condition) and then comparing scores. A higher score indicates a lower degree of neurological disorder. The results are illustrated in FIG. 2. The vertical axis in FIG. 2 indicates the score for the degree of neurological disorder.

As illustrated in FIG. 2, 24 hours after reperfusion, the hemoglobin-albumin complex-administered group of Example 1 had a high score of 12.9 ± 0.5, whereas the control group of Comparative Example 1 had a score of 8.6 ± 0.7, and a statistically significant effect of symptom improvement was confirmed (FIG. 2).

### (4) Pathological evaluation

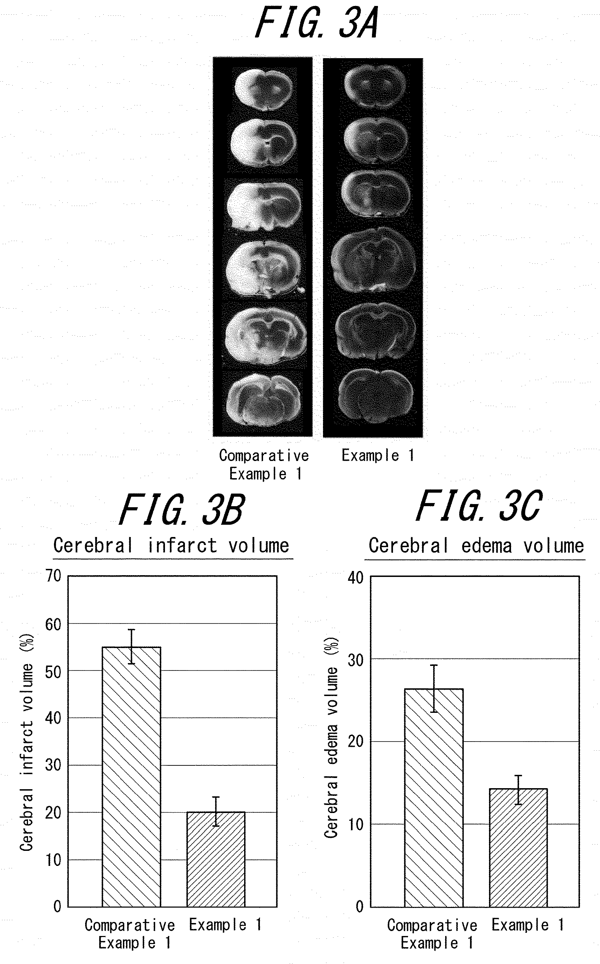

In the pathological evaluation, brains sections were stained using 2,3,5-triphenyltetrazolium chloride (TTC) dye, cerebral infarct area in serial sections and cerebral edema area in serial sections were measured, and cerebral infarct volume (%) and cerebral edema volume (%) were calculated. Larger cerebral infarct volume and cerebral edema volume indicate greater injury due to cerebral infarction. The results are illustrated in FIGS. 3A to 3C. FIG. 3A shows TTC stained photographs of brain coronal plane sections arranged in order of the coronal planes from anterior to posterior. The vertical axes in FIGS. 3B and 3C respectively indicate cerebral infarct volume (%) and cerebral edema volume (%).

As illustrated in FIG. 3B, the cerebral infarct volume 24 hours after reperfusion was reduced to 20.2 ± 3.1% for the hemoglobin-albumin complex-administered group of Example 1 relative to 55.2 ± 3.6% for the control group of Comparative Example 1 (i.e., reduced to 37% of the control group value), and a statistically significant reductive effect was observed. Moreover, the hemoglobin-albumin complex-administered group also had a smaller cerebral edema volume of 14.1 ± 1.75% relative to a cerebral edema volume of 26.4 ± 2.8% for the control group, and a statistically significant reductive effect was confirmed (FIG. 3C).

### (5) Molecular biological evaluation

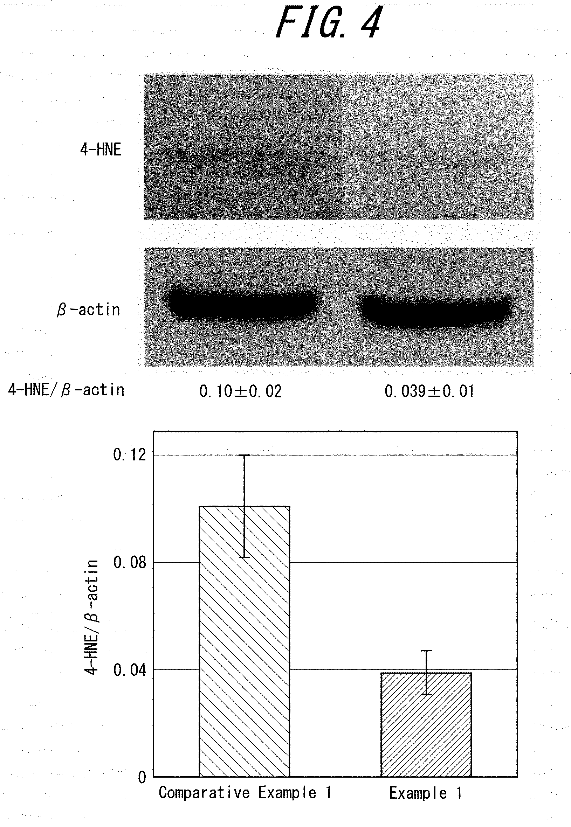

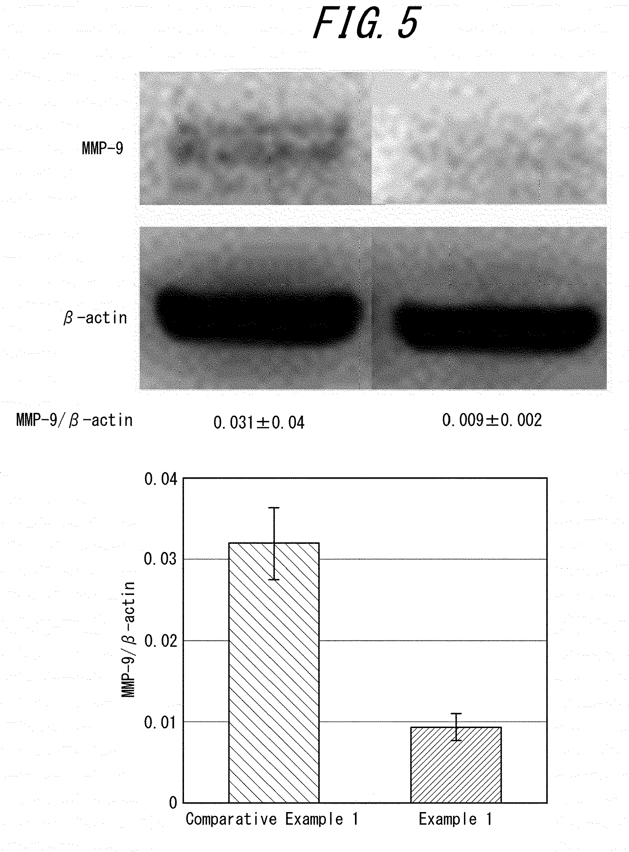

Molecular biological evaluation was performed by quantifying 4-HNE abundance and MMP-9 expression by western blotting using brain tissue as a sample. The amount of β-actin in the sample was measured, and ratios of 4-HNE abundance/β-actin expression and MMP-9 expression/β-actin expression were determined. Quantification of abundance and expression was performed by densitometry. Higher 4-HNE abundance indicates that tissue has been affected by oxidative stress and higher MMP-9 expression indicates that inflammatory condition has been promoted. The results are illustrated in FIGS.4A, 4B, 5A, and 5B.

As illustrated in FIGS. 4A, 4B, 5A, and 5B, 4-HNE abundance and MMP-9 expression were found to be significantly reduced in the hemoglobin-albumin complex-administered group of Example 1. These results suggest that administration of the hemoglobin-albumin complex inhibits production of active oxygen species such as reactive oxygen species (ROS) and also suggest that administration of the hemoglobin-albumin complex suppresses inflammatory response. Accordingly, the expression of a tissue-protecting effect through administration of the hemoglobin-albumin complex is strongly suggested from a molecular biological viewpoint (FIGS. 4A, 4B, 5A, and 5B).

From the results described above, it was possible to confirm a significant improvement in neurological symptoms and a significant reduction of cerebral infarct volume and cerebral edema volume for the hemoglobin-albumin complex-administered group compared to the control group. Moreover, the results demonstrate that 4-HNE abundance and MMP-9 expression were significantly reduced in the hemoglobin-albumin complex-administered group. The hemoglobin-albumin complex is thought to inhibit endothelial cell damage, and ultimately inhibit production of active oxygen species and expression of MMP-9 to display a nerve/blood vessel-protecting effect as a result of being able to effectively transport oxygen to narrowed microvessels.

As has been described above, ischemia-reperfusion injury is reduced and a tissue-protecting effect is obtained through administration of the therapeutic agent according to the present disclosure in a disease model of cerebral infarction, which is one type of ischemic disease. This provides evidence that the therapeutic agent according to the present disclosure is useful in treatment of ischemic disease. Although Example 1 provides an example in which a disease model of cerebral infarction is adopted, ischemic disease is not limited to the brain and progresses by the same mechanism in other organs. Therefore, the therapeutic agent according to the present disclosure can also provide a good therapeutic effect in ischemic disease other than ischemic disease in the brain.

Next, the effect of the hemoglobin-albumin complex on the swelling of astrocytes around microvessels that occurs during the hyperacute phase of reperfusion was verified. Transient MCAO model rats were prepared and a reperfusion condition was induced therein after 2 hours of ischemia. Rats were euthanized after 15 minutes, after 2 hours, and after 6 hours (i.e., during the hyperacute phase of reperfusion), and their brains were excised. Perfusion condition of the hemoglobin-albumin complex in microvessels and morphological change of microvessels were evaluated. Separate rats were used to evaluate brain tissue oxygen partial pressure and cerebral blood flow prior to MCAO surgery, directly after surgery, directly prior to reperfusion, directly after reperfusion, 2 hours after reperfusion, and 6 hours after reperfusion with the rats still alive. Note that ischemia, reperfusion, and drug administration were carried out by the same methods as previously described in "(2) Ischemia reperfusion test", and a hemoglobin-albumin complex-administered group (Example 1) and a control group (Comparative Example 1) were compared.

### (6) Evaluation of perfusion condition in microvessels

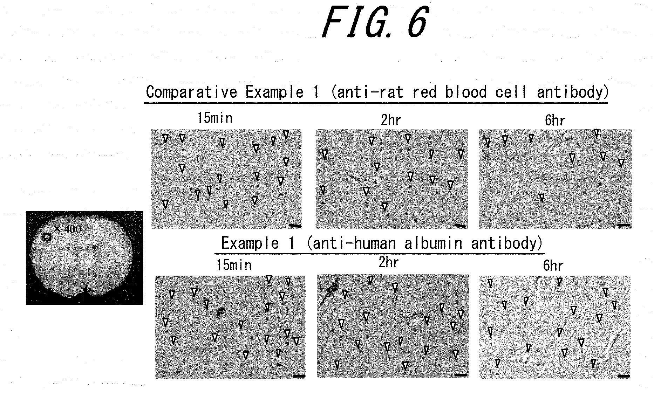

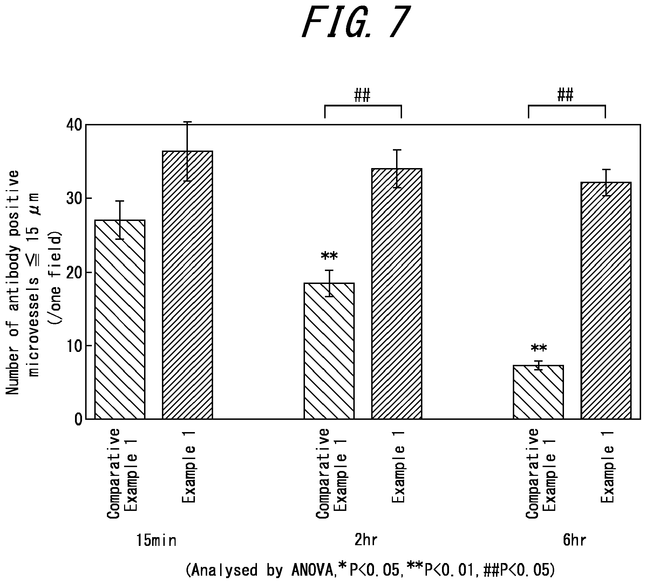

Paraffin sections of the excised brains were prepared, and the perfusion condition of the hemoglobin-albumin complex and rat red blood cells in a reperfused region during the hyperacute phase of reperfusion was quantified through immunohistochemical staining. The hemoglobin-albumin complex was detected in the hemoglobin-albumin complex-administered group using anti-human albumin antibody and rat red blood cells were detected in the control group using anti-rat red blood cell antibody. Quantification was carried out by counting the number of microvessels of 15 µm or less in diameter in the reperfused region that were positive for the respective antibodies. The results are illustrated in FIGS. 6 and 7. FIG. 6 shows microscope observations made at ×400 magnification. The scale bars in FIG. 6 indicate 20 µm. Black arrowheads indicate microvessels that are antibody positive. FIG. 7 compares, as a bar graph, the counted number of microvessels that are antibody positive in one field of view (×400) for the control group and the hemoglobin-albumin complex-administered group. The number of microvessels to which rat red blood cells perfused significantly decreased over time for 15 minutes, 2 hours, and 6 hours after reperfusion. On the other hand, the number of microvessels to which the hemoglobin-albumin complex perfused did not change over time for 15 minutes, 2 hours, and 6 hours after reperfusion, which indicates that perfusion is maintained in microvessels even as time passes.

### (7) Morphological change of microvessels

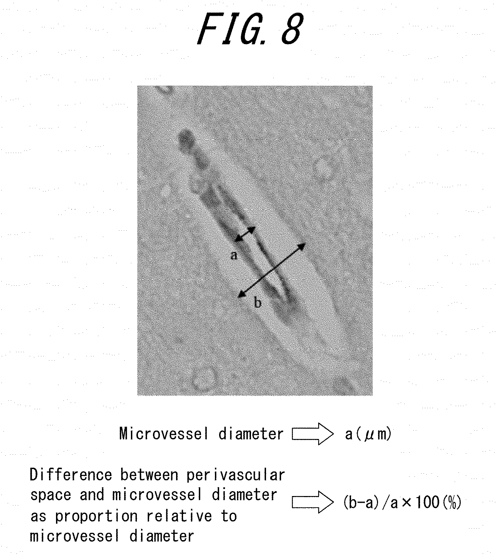

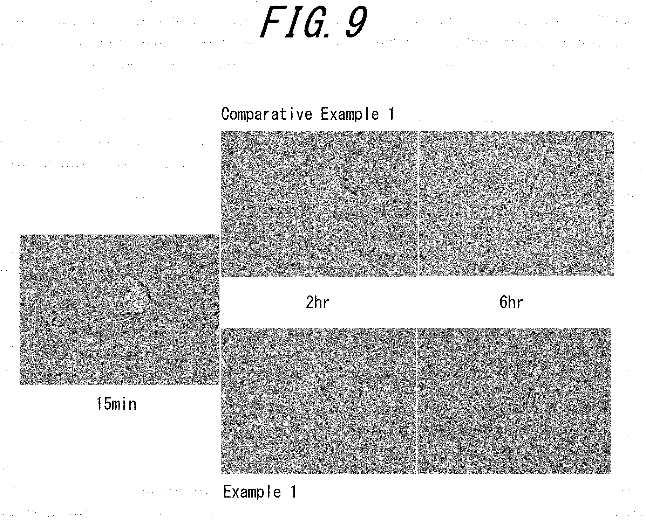

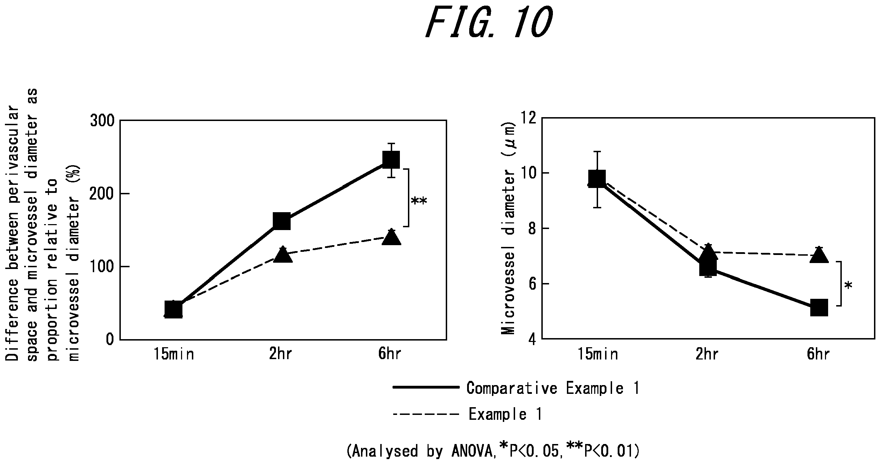

Paraffin sections of excised brains were prepared in the same way as in evaluation of perfusion of the hemoglobin-albumin complex. The change in vessel internal diameter of microvessels in a reperfused region during the hyperacute phase of reperfusion was quantified through immunohistochemical staining. Vascular endothelium cells were immunostained using anti-von Willebrand factor antibody, the internal diameter of blood vessels (microvessel diameter) and the length of perivascular space were measured (FIG. 8), and a hemoglobin-albumin complex-administered group and a control group were compared. FIG. 8 illustrates a method by which a difference between perivascular space and microvessel diameter is measured as a proportion (%) relative to microvessel diameter. Microvessels having a vessel internal diameter of 15 µm or less were used as measurement targets. The results are illustrated in FIGS. 9 and 10. FIG. 9 shows microscope observations made at ×400 magnification. In the control group, narrowing of microvessel internal diameter and an increase in the difference between perivascular space and microvessel diameter as a proportion relative to microvessel diameter were confirmed to occur over time at 2 hours and 6 hours after reperfusion, and blood vessel narrowing due to perivascular space swelling during the hyperacute phase of reperfusion was confirmed as illustrated in FIG. 10. On the other hand, in the hemoglobin-albumin complex-administered group, significant inhibition of narrowing of blood vessel diameter and suppression of an increase in the difference between perivascular space and microvessel diameter as a proportion relative to microvessel diameter at 6 hours after reperfusion were confirmed compared to the control group, which suggests that the hemoglobin-albumin complex inhibits swelling of perivascular astrocytes.

### (8) Brain tissue partial pressure and cerebral blood flow

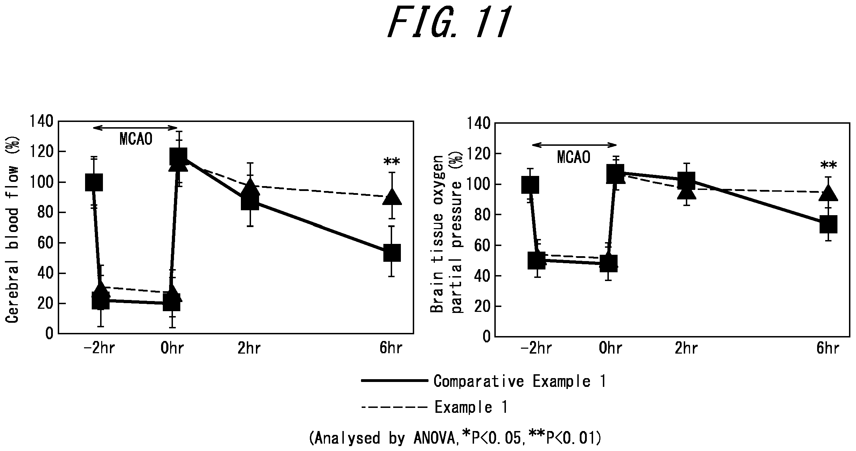

Changes in brain tissue oxygen partial pressure and cerebral blood flow in a reperfused region during the hyperacute phase of reperfusion (directly prior to MCAO, directly after MCAO, directly prior to reperfusion, directly after reperfusion, 2 hours after reperfusion, and 6 hours after reperfusion) were measured and quantified. Measurement of brain tissue oxygen partial pressure was carried out using a tissue oxygen partial pressure meter (POG-203, Unique Medical Co., Ltd., Tokyo). A small hole was punctured in the skull of a rat at a location 3 mm posteriorly and 3 mm laterally to the right from the bregma (intersection of sagittal suture and coronal suture), an oxygen electrode was inserted at a position 3 mm from the brain surface, and tissue oxygen partial pressure was measured in the penumbra. The cerebral blood flow was measured using the previously described laser blood flowmeter (FLO-C1, Omegawave) by applying a probe over the dura mater of a right middle cerebral artery region. The results are illustrated in FIG. 11. In the control group, the cerebral blood flow and brain tissue oxygen partial pressure dropped rapidly from 2 hours after reperfusion to 6 hours after reperfusion. Moreover, it was confirmed that cerebral blood flow and brain tissue oxygen partial pressure in the penumbra 6 hours after reperfusion decreased significantly in the control group compared to in the hemoglobin-albumin complex-administered group. These results indicate that in the hemoglobin-albumin complex-administered group, cerebral blood flow and brain tissue oxygen partial pressure in the penumbra were maintained, and reduction thereof was inhibited during the hyperacute phase of reperfusion.

From the results described above, it was possible to confirm sustained favorable perfusion of the hemoglobin-albumin complex by microcirculation, inhibition of microvessel narrowing, and maintenance of cerebral blood flow and brain tissue oxygen partial pressure in the penumbra during the hyperacute phase of reperfusion in the hemoglobin-albumin complex-administered group compared to the control group. Narrowing of microvessels due to astrocyte swelling during the hyperacute phase of reperfusion is considered to be an important condition that exacerbates ischemia-reperfusion injury. The hemoglobin-albumin complex is thought to inhibit narrowing of microvessels and maintain cerebral blood flow during the hyperacute phase of reperfusion to thereby enable transportation of oxygen and improvement in the ischemic condition of brain tissue.

### (Example 2 and Comparative Example 2)

### (1) Production of therapeutic agent

### -Production Example 4: Production of therapeutic agent B-

A therapeutic agent B containing liposome-encapsulated hemoglobin (LEH) was produced by the following method.

Water (336 g) was added to uniformly mixed lipid composed of 182 g of hydrogenated soybean phosphatidylcholine, 89 g of cholesterol, and 65 g of stearic acid, and was heated therewith at 80°C for 30 minutes to produce hydrated, swollen, and uniformly mixed lipid. Hemoglobin was purified and concentrated from an out-of-date packed red blood cell formulation. An equivalent number of moles of inositol hexaphosphate was added to the hemoglobin to produce concentrated, high-purity hemoglobin solution having a hemoglobin concentration of 45 w/w%. Next, 2,688 g of the concentrated, high-purity hemoglobin solution was added to 672 g of the hydrated, swollen, and uniformly mixed lipid, and emulsification was carried out by high-speed stirring at a temperature of 45°C or lower to obtain a mixed emulsion of hemoglobin-containing liposomes. The emulsion was diluted using saline, and the particle diameter was controlled through circulation filtration using a membrane having a pore diameter of 0.45 µm. In addition, a circulation filtration system operating through ultrafiltration with a cutoff molecular weight of 300,000 was used to remove inositol hexaphosphate and hemoglobin not contained in the liposomes and perform concentration through a water-addition and concentration operation using saline to obtain a suspension of hemoglobin-containing liposomes suspended in saline.

The hemoglobin-containing liposome suspension was added to a saline solution of polyethylene glycol (PEG)-phosphatidylethanolamine to obtain a suspension (therapeutic agent B) of hemoglobin-containing PEG-modified liposomes suspended in saline having a hemoglobin concentration of 6 g/dL.

The concentrations of hemoglobin content in the therapeutic agents A and B, the particle diameters of the hemoglobin-albumin complex and LEH, the surface charges of the hemoglobin-albumin complex and LEH, and the oxygen saturations of the hemoglobin-albumin complex and LEH are shown in Table 1.

**Table 1**

| | Therapeutic agent A | Therapeutic agent B |
|---|---|---|
| Hb concentration | 5 g/dL | 6 g/dL |
| Particle diameter | 10 nm | 200-250 nm |
| Surface charge | Negative | Neutral |
| Oxygen affinity | 9 Torr | 30 Torr |

### (2) Ischemia reperfusion test

Eight to nine week-old rats (male Sprague-Dawley Rats (SD Rats) weighing 280 g to 320 g) were each anesthetized by isoflurane inhalation. A filament embolus (silicone-coated 4-0 nylon filament) was inserted from the internal carotid artery, was advanced upward until the origin of the middle cerebral artery was occluded, and was secured in place to create an ischemic condition in the middle cerebral artery region. Reduction in cerebral blood flow in ischemia was checked using a laser blood flowmeter (FLO-C1, Omegawave). Note that only rats for which the blood flow had decreased to 30% of the former value were used in subsequent experimentation. After two hours of ischemia, each of the rats was anesthetized once again, and the filament embolus was withdrawn to create a reperfusion condition. In the reperfusion, a tube (PE10 tube) for infusion was inserted into the internal carotid artery into which the filament embolus had been inserted, and the therapeutic agent A or B was intra-arterially administered under conditions shown in Table 2 to the region in which ischemia had occurred (Example 2 and Comparative Example 2). A group for which the filament embolus was withdrawn without administration of a therapeutic agent was used as a control group.

The rats were euthanized 24 hours after reperfusion and their brains were excised and used in pathological evaluation by TTC staining. The cerebral infarct area of rats in Example 2 and Comparative Example 2 were each measured as a proportion relative to the cerebral infarct area of rats in the control group to which a therapeutic agent was not administered, which was taken to be 100%. A lower relative proportion of cerebral infarct area indicates reduction of ischemic injury to the brain. The results are shown in Table 2.

**Table 2**

| | Example 2 | Comparative Example 2 |
|---|---|---|
| Type of therapeutic agent | Therapeutic agent A | Therapeutic agent B |
| Dose | 2 mL (80 mL/kg/h) | 6 mL (80 mL/kg/h) |
| Administration time | 5 mins | 15 mins |
| Relative proportion of cerebral infarct area | 36.6% | 59.9% |

Upon comparison of Example 2 and Comparative Example 2, administration of the therapeutic agent A (therapeutic agent according to the present disclosure) to transient middle cerebral artery occlusion model rats enabled significant reduction in tissue damage due to infarction relative to administration of the therapeutic agent B having LEH as an active ingredient.

### INDUSTRIAL APPLICABILITY

According to the present disclosure, it is possible to provide a therapeutic agent for ischemic disease that reduces ischemia-reperfusion injury and displays a tissue-protecting effect.

### REFERENCE SIGNS LIST

- 10: hemoglobin
- 20: albumin
- 100: hemoglobin-albumin complex

## Claims

1. A therapeutic agent for ischemic disease comprising, as an active ingredient, a hemoglobin-albumin complex in which albumin serving as a shell is bound to hemoglobin serving as a core via a crosslinker.

2. The therapeutic agent for ischemic disease according to claim 1, wherein the ischemic disease is at least one selected from the group consisting of ischemic cerebrovascular disease, ischemic heart disease, ischemic lung disease, ischemic nephropathy, ischemic hepatitis, and limb ischemia.

3. The therapeutic agent for ischemic disease according to claim 1, wherein the ischemic disease is cerebral infarction.

4. The therapeutic agent for ischemic disease according to any one of claims 1 to 3, administered in order to deliver oxygen to tissue to which oxygen cannot be delivered by red blood cells.

5. The therapeutic agent for ischemic disease according to any one of claims 1 to 3, used not in combination with treatment for relieving vascular occlusion or vascular constriction.

6. The therapeutic agent for ischemic disease according to any one of claims 1 to 3, used in combination with treatment for relieving vascular occlusion or vascular constriction.

7. The therapeutic agent for ischemic disease according to claim 6, used to reduce ischemia-reperfusion injury through intravascular administration when recanalization therapy is performed or after ischemia reperfusion.

8. The therapeutic agent for ischemic disease according to claim 3, wherein, in a situation in which an individual who has undergone normal reperfusion and an individual who has been administered the therapeutic agent according to claim 3 in combination with reperfusion are compared in terms of cerebral infarct volume and cerebral edema volume, either or both of cerebral infarct volume and cerebral edema volume are significantly smaller for the individual who has been administered the therapeutic agent according to claim 3 than for the individual who has undergone normal reperfusion.

9. The therapeutic agent for ischemic disease according to claim 3, wherein, in a situation in which an individual who has undergone normal reperfusion and an individual who has been administered the therapeutic agent according to claim 3 in combination with reperfusion are compared in terms of abundance of 4-HNE and expression of matrix metalloprotease-9 at a cerebral infarction site, either or both of abundance of 4-HNE and expression of matrix metalloprotease-9 are significantly lower for the individual who has been administered the therapeutic agent according to claim 3 than for the individual who has undergone normal reperfusion.

10. The therapeutic agent for ischemic disease according to claim 3, wherein, in a situation in which an individual who has undergone normal reperfusion and an individual who has been administered the therapeutic agent according to claim 3 in combination with reperfusion are compared in terms of post-cerebral infarction neurological disorder, neurological disorder is significantly improved for the individual who has been administered the therapeutic agent according to claim 3 relative to the individual who has undergone normal reperfusion.

11. The therapeutic agent for ischemic disease according to any one of claims 1 to 10, wherein a single dose of the therapeutic agent to an individual is 100 mg to 1,000 mg, in terms of hemoglobin content, per 1 kg of body weight of the individual.

12. The therapeutic agent for ischemic disease according to any one of claims 1 to 11, wherein the hemoglobin-albumin complex has an average particle diameter of 30 nm or less.

13. The therapeutic agent for ischemic disease according to any one of claims 1 to 12, wherein a bonding site with the crosslinker in the hemoglobin of the hemoglobin-albumin complex is lysine, and a bonding site with the crosslinker in the albumin of the hemoglobin-albumin complex is cysteine 34.

14. The therapeutic agent for ischemic disease according to claim 13, wherein a bond between the hemoglobin and the crosslinker is an amide bond, and a bond between the albumin and the crosslinker is a sulfide bond or a disulfide bond.

15. The therapeutic agent for ischemic disease according to any one of claims 1 to 14, wherein the number of molecules of the albumin in the hemoglobin-albumin complex is 1 to 7.

16. The therapeutic agent for ischemic disease according to any one of claims 1 to 15, wherein the hemoglobin is at least one selected from the group consisting of human hemoglobin, bovine hemoglobin, pig hemoglobin, horse hemoglobin, dog hemoglobin, cat hemoglobin, rabbit hemoglobin, recombinant human hemoglobin, and intramolecularly crosslinked hemoglobin.

17. The therapeutic agent for ischemic disease according to any one of claims 1 to 16, wherein the albumin is at least one selected from the group consisting of human serum albumin, bovine serum albumin, pig serum albumin, horse serum albumin, dog serum albumin, cat serum albumin, rabbit serum albumin, recombinant human serum albumin, recombinant bovine serum albumin, recombinant dog serum albumin, and recombinant cat serum albumin.

18. The therapeutic agent for ischemic disease according to claim 14, wherein the crosslinker is at least one selected from the group consisting of compounds represented by general formulae (1) to (3) and chemical formula (1), shown below, 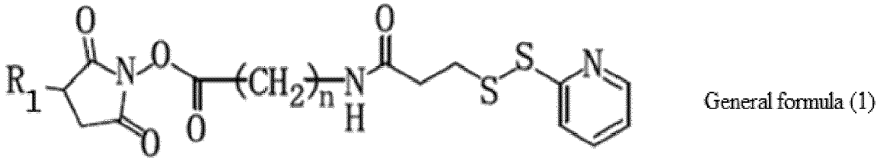 where, in general formula (1), R₁ represents a hydrogen atom or SO₃⁻Na⁺ and n represents an integer of 1 to 10, 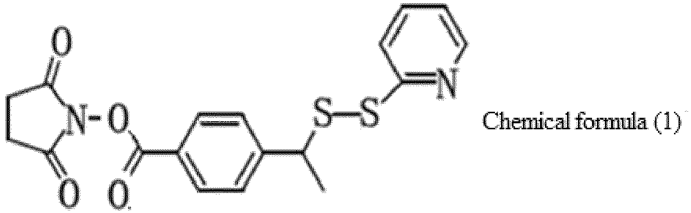 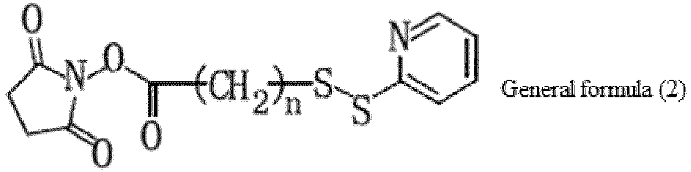 in general formula (2), n represents an integer of 1 to 10, 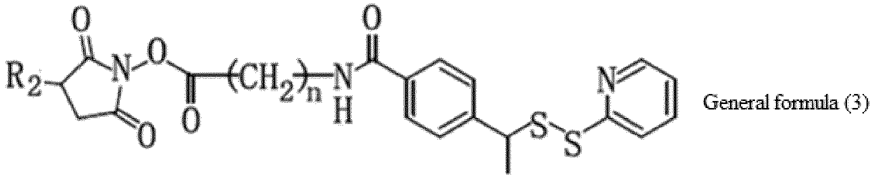 and in general formula (3), R₂ represents a hydrogen atom or SO₃⁻Na⁺ and n represents an integer of 1 to 10.

19. The therapeutic agent for ischemic disease according to claim 14, wherein the crosslinker is a compound represented by general formula (4), shown below, 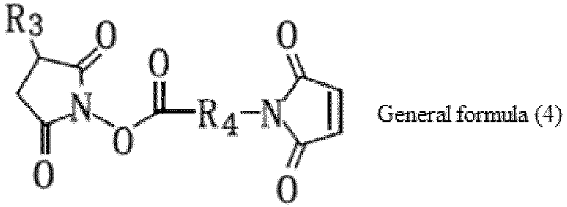 where, in general formula (4), R₃ represents a hydrogen atom or SO₃⁻Na⁺ and R₄ represents any one of general formulae (5) and (6) and chemical formulae (2) to (4), shown below, 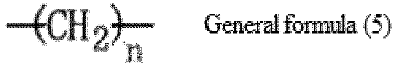 in general formula (5), n represents an integer of 1 to 10, 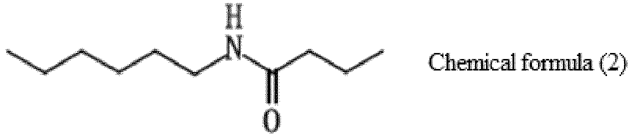 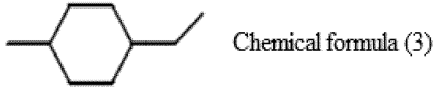 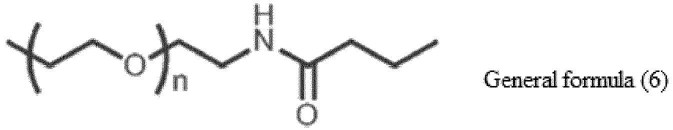 and in general formula (6), n represents an integer of 2, 4, 6, 8, 10, or 12,
